# EUROPEAN PATENT SPECIFICATION

(11) **EP 4 042 990 B1**
(45) Date of publication and mention of the grant of the patent: **28.10.2026**
(21) Application number: 22166676.1
(22) Date of filing: 16.12.2015
(51) Int. Cl.: A61F 9/007, A61M 1/00, A61F 9/008, A61M 3/02

(54) **SURGICAL CASSETTE SYSTEM**
SYSTEM FÜR CHIRURGISCHE KASSETTEN
SYSTÈME DE CASSETTE CHIRURGICALE

(30) Priority: 14.04.2015 US 201514686582
(43) Date of publication of application: 17.08.2022
(60) Divisional application: 26183877.5 / 4 799 661
(62) Document number(s) of the earlier application(s) in accordance with Art. 76 EPC: 20156719.5 / 3 682 914; 15823070.6 / 3 283 138
(73) Proprietor: Johnson & Johnson Surgical Vision, Inc., Irvine, CA 92618 (US)
(72) Inventor: ROSS, Mark, W., Costa Mesa, 92627 (US); GERG, James, B., Lake Forest, 92630 (US)
(74) Representative: Carpmaels & Ransford LLP

(56) References cited:
- WO-A1-2009/123547
- WO-A1-2012/151062
- WO-A2-2007/143677
- US-A1- 2013 245 543

## Description

### TECHNICAL FIELD OF THE INVENTION

The present invention is generally related to systems for controlling surgical fluid flows, particularly during treatment of an eye.

### BACKGROUND OF THE INVENTION

The optical elements of the eye include both a cornea (at the front of the eye) and a lens within the eye. The lens and cornea work together to focus light onto the retina at the back of the eye. The lens also changes in shape, adjusting the focus of the eye to vary between viewing near objects and far objects. The lens is found just behind the pupil, and within a capsular bag. This capsular bag is a thin, relatively delicate structure which separates the eye into anterior and posterior chambers.

With age, clouding of the lens or cataracts are fairly common. Cataracts may form in the hard central nucleus of the lens, in the softer peripheral cortical portion of the lens, or at the back of the lens near the capsular bag.

Cataracts can be treated by the replacement of the cloudy lens with an artificial lens. Phacoemulsification systems often use ultrasound energy to fragment the lens and aspirate the lens material from within the capsular bag. This may allow the capsular bag to be used for positioning of the artificial lens, and maintains the separation between the anterior portion of the eye and the vitreous humour in the posterior chamber of the eye.

During cataract surgery and other therapies of the eye, accurate control over the volume of fluid within the eye is highly beneficial. For example, while ultrasound energy breaks up the lens and allows it to be drawn into a treatment probe with an aspiration flow, a corresponding irrigation flow may be introduced into the eye so that the total volume of fluid in the eye does not change excessively. If the total volume of fluid in the eye is allowed to get too low at any time during the procedure, the eye may collapse and cause significant tissue damage. Similarly, excessive pressure within the eye may strain and injure tissues of the eye.

While a variety of specific fluid transport mechanisms have been used in phacoemulsification and other treatment systems for the eyes, aspiration flow systems can generally be classified in two categories: 1) volumetric-based aspiration flow systems using positive displacement pumps; and 2) vacuum-based aspiration systems using a vacuum source, typically applied to the aspiration flow through an air-liquid interface. These two categories of aspiration flow systems each have unique characteristics that render one more suitable for some procedures than the other, and vice versa.

Among positive displacement aspiration systems, peristaltic pumps (which use rotating rollers that press against a flexible tubing to induce flow) are commonly employed. Such pumps provide accurate control over the flow volume. The pressure of the flow, however, is less accurately controlled and the variations in vacuum may result in the feel or traction of the handpiece varying during a procedure. Peristaltic and other displacement pump systems may also be somewhat slow.

Vacuum-based aspiration systems provide accurate control over the fluid pressure within the eye, particularly when combined with gravity-fed irrigation systems. While vacuum-based systems can result in excessive fluid flows in some circumstances, they provide advantages, for example, when removing a relatively large quantity of the viscous vitreous humour from the posterior chamber of the eye. However, Venturi pumps and other vacuum-based aspiration flow systems arc subject to pressure surges during occlusion of the treatment probe, and such pressure surges may decrease the surgeon's control over the eye treatment procedure.

Different tissues may be aspirated from the anterior chamber of the eye with the two different types of aspiration flow. For example, vacuum-induced aspiration flow may quickly aspirate tissues at a significant distance from a delicate structure of the eye (such as the capsular bag), while tissues that are closer to the capsular bag are aspirated more methodically using displacement-induced flows.

Conventionally, fluid aspiration systems include a console and a fluidic cassette mounted on the console. The fluidic cassette is typically changed for each patient and cooperates with the console to provide fluid aspiration. Generally, a single type of cassette is used by a particular console, regardless of whether the procedure will require positive displacement aspiration, vacuum-based aspiration, or both. U.S. Patent 8,070,712; U.S. Published Application 2008011431; and U.S. Published Application 20080114291 provide examples of cassettes. U.S. Application 13/776, 988 provides examples of cassettes. Further, WO 2007/143677 shows a cassette for use with an ophthalmic surgical pump for collecting aspirant fluid and tissue from a patient's eye includes a rigid walled container having an interior volume. At least one tapered alignment slot is formed in a side wall of the container and extends from a back wall towards a front wall. An irrigation and aspiration manifold base is removeably attached to the container. An aspiration path is formed within the container for receiving the aspiration fluid and the tissue from the eye and directing the flow of fluid towards a front half of the container before the fluid and tissue collects within a majority of the interior volume of the container. A fluid level indicator is formed on a wall of the container, such that an associated photo-detector of the pump may determine a level of fluid in the container.

In light of the above, it would be advantageous to provide improved devices, systems, and methods for eye surgery.

### SUMMARY OF THE INVENTION

The present invention provides a surgical system, including a cassette having a front housing, a rear housing, and a reservoir, wherein the front housing has one or more molded fluid channels, and wherein the front housing, rear housing, and reservoir are made of a transparent material; a console having a cassette receptacle configured and dimensioned to receive the cassette, wherein the cassette receptacle has a light, wherein when in operation, the light is configured to illuminate the cassette enabling visualization of fluid flow through the molded fluid channels and/or into the reservoir. Further, the system includes a cassette detector, wherein the cassette detector is configured to determine the pumping functionality of the cassette. The pumping functionality may be selected from the group consisting of peristaltic, Venturi, or both. The cassette detector may be a reflective object sensor, a photo interrupter sensor, ultrasonic, a laser distance sensor, a bar code sensor, or a pattern recognition sensor. The invention is defined in the claims. Other systems, devices, methods, embodiments, examples, items etc., to the extent that they are not a part of the claimed invention, are provided as an aid to the skilled person's understanding of the invention.

### BRIEF DESCRIPTION OF THE DRAWINGS

The present invention is best understood with reference to the following detailed description of the disclosure and the drawings in which:
FIG. 1 schematically illustrates an eye treatment system in which a cassette couples an eye treatment probe with an eye treatment console;
FIGs. 2A and 2B are exploded views of an exemplary surgical cassette manifold for use in the system of Fig. 1;
FIG. 3A is perspective front view of the front housing of an exemplary surgical cassette manifold;
FIG. 3B is perspective back view of the front housing of an exemplary surgical cassette manifold;
FIG. 4A is a front perspective view of the rear housing of an exemplary surgical cassette manifold;
FIG. 4B is a back perspective view of the rear housing of an exemplary surgical cassette manifold;
FIG. 4C is front perspective view of the rear housing of an exemplary surgical cassette manifold;
FIG. 4D is a back perspective view of the rear housing of an exemplary surgical cassette manifold;
FIG. 4E is front perspective view of the rear housing of an exemplary surgical cassette manifold;
FIG. 4F is a back perspective view of the rear housing of an exemplary surgical cassette manifold;
FIG. 4G is a back perspective view of an assembled exemplary surgical cassette having rear housing shown in FIGs. 4E and 4F;
FIG. 5A is back view of the rear housing of an exemplary surgical cassette manifold;
FIG. 5B is a cross-sectional view of the rear housing along N-N of an exemplary surgical cassette manifold;
FIG. 5C is a cross-sectional view of the rear housing of an exemplary surgical cassette manifold;
FIG. 5D is a larger view of a portion of cross-sectional view of the rear housing of the exemplary surgical cassette manifold shown in FIG. 5C; and
FIG. 6 is a perspective view of an exemplary surgical cassette with attached tubing and drain bag.

### DETAILED DESCRIPTION OF THE INVENTION

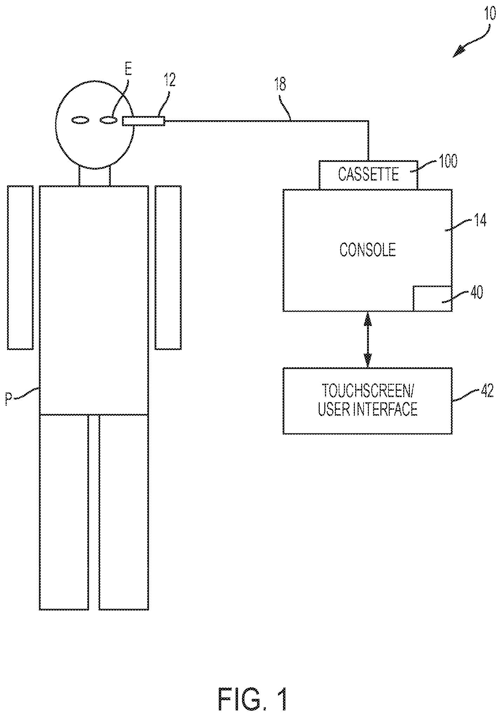

Referring to FIG. 1, a system 10 for treating an eye E of a patient P generally includes an eye treatment probe handpiece 12 coupled to a console 14 by a cassette 100 mounted on the console. Handpiece 12 may include a handle for manually manipulating and supporting an insertable probe tip. The probe tip has a distal end which is insertable into the eye, with one or more lumens in the probe tip allowing irrigation fluid to flow from the console 14 and/or cassette 100 into the eye. Aspiration fluid may also be withdrawn through a lumen of the probe tip, with the console 14 and cassette 100 generally including a vacuum aspiration source, a positive displacement aspiration pump, or both to help withdraw and control a flow of surgical fluids into and out of eye E. As the surgical fluids may include biological materials that should not be transferred between patients, cassette 100 will often comprise a disposable (or alternatively, re-sterilizable) structure, with the surgical fluids being transmitted through flexible conduits 18 of the cassette that avoid direct contact in between those fluids and the components of console 14.

When a distal end of the probe tip of handpiece 12 is inserted into an eye E, for example, for removal of a lens of a patient with cataracts, an electrical conductor and/or pneumatic line (not shown) may supply energy from console 14 to an ultrasound transmitter of the handpiece, a cutter mechanism, or the like. Alternatively, the handpiece 12 may be configured as an irrigation/aspiration (I/A) or vitrectomy handpiece. Also, the ultrasonic transmitter may be replaced by other means for emulsifying a lens, such as a high energy laser beam. The ultrasound energy from handpiece 12 helps to fragment the tissue of the lens, which can then be drawn into a port of the tip by aspiration flow. So as to balance the volume of material removed by the aspiration flow, an irrigation flow through handpiece 12 (or a separate probe structure) may also be provided, with both the aspiration and irrigations flows being controlled by console 14.

So as to avoid cross-contamination between patients without incurring excessive expenditures for each procedure, cassette 100 and its flexible conduit 18 may be disposable. Alternatively, the flexible conduit or tubing may be disposable, with the cassette body and/or other structures of the cassette being sterilizable. Regardless, the disposable components of the cassette are typically configured for use with a single patient, and may not be suitable for sterilization. The cassette will interface with reusable (and often quite expensive) components of console 14, which may include one or more peristaltic pump rollers, a Venturi or other vacuum source, a controller 40, and the like.

Controller 40 may include an embedded microcontroller and/or many of the components common to a personal computer, such as a processor, data bus, a memory, input and/or output devices (including a touch screen user interface 42), and the like. Controller 40 will often include both hardware and software, with the software typically comprising machine readable code or programming instructions for implementing one, some, or all of the methods described herein. The code may be embodied by a tangible media such as a memory, a magnetic recording media, an optical recording media, or the like. Controller 40 may have (or be coupled to) a recording media reader, or the code may be transmitted to controller 40 by a network connection such as an internet, an intranet, an Ethernet, a wireless network, or the like. Along with programming code, controller 40 may include stored data for implementing the methods described herein, and may generate and/or store data that records perimeters with corresponding to the treatment of one or more patients. Many components of console 14 may be found in or modified from known commercial phacoemulsification systems from Abbott Medical Optics Inc. of Santa Ana, California; Alcon Manufacturing, Ltd. of Ft. Worth, Texas; Bausch and Lomb of Rochester, New York; and other suppliers.

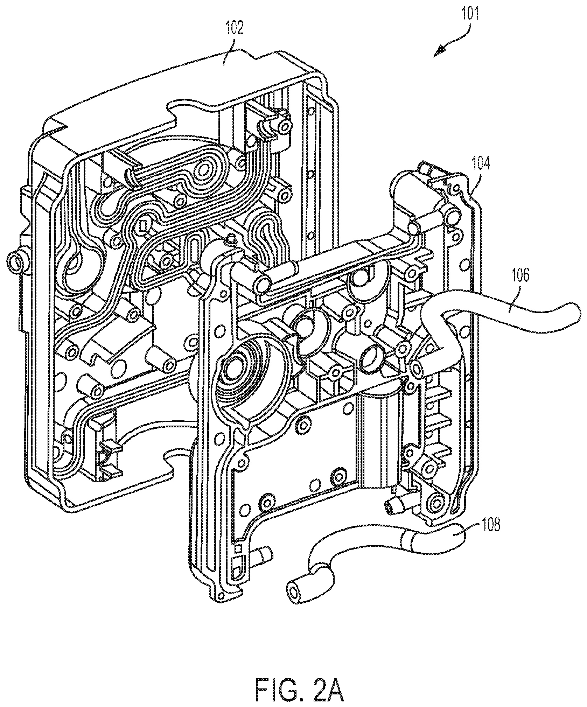

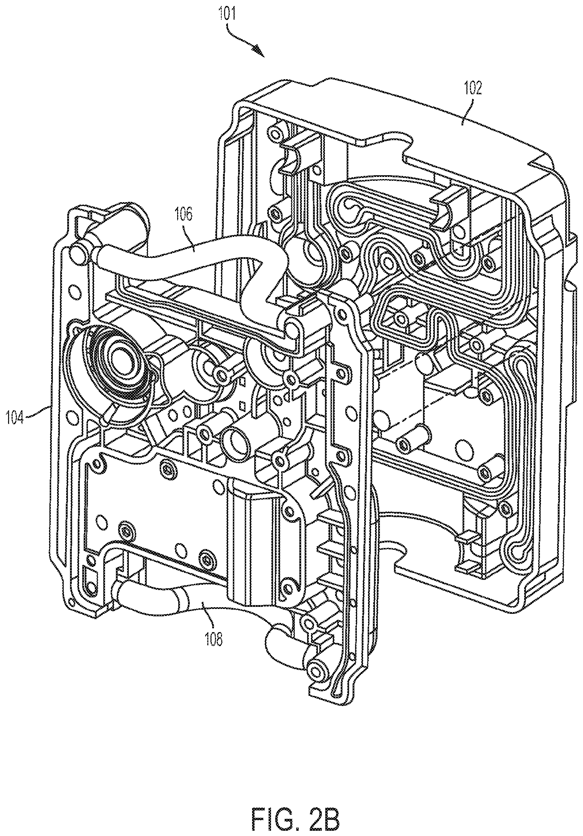

FIGs. 2A and 2B illustrates a surgical cassette manifold of the present invention, including components of surgical cassette manifold 101. Cassette or surgical cassette 100 is an assembly surgical cassette manifold 101 having fluid pathways and connected tubing configured to manage one or more of the following: fluid inflow, fluid outflow, fluid vacuum level, and fluid pressure in a patient's eye E when coupled with console 14. As shown in FIGs. 2A and 2B, surgical cassette manifold 100 has a front housing 102, a rear housing 104, a first tubing 106, and a second tubing 108. Rear housing 104 may also have gasket 110 co-molded or over-molded with rear housing 104.

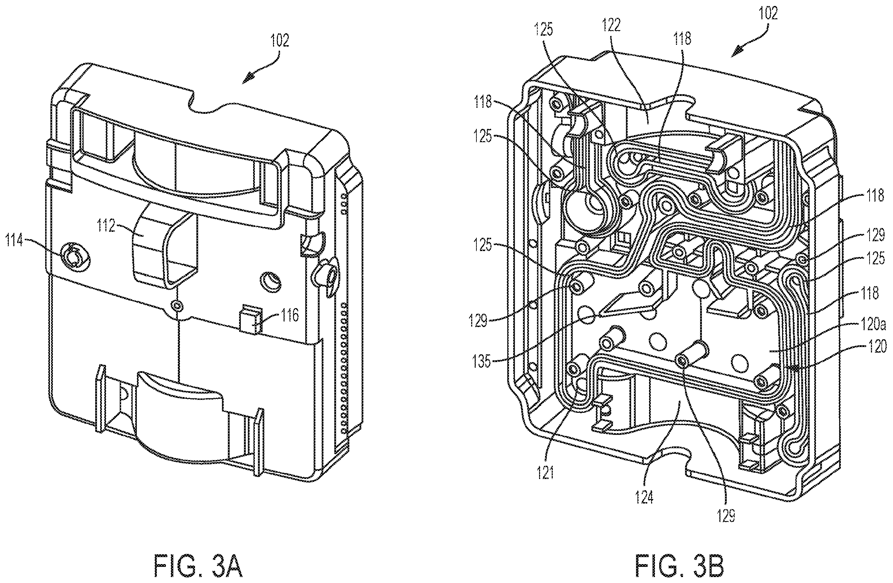

FIG. 3A and 3B show front housing 102 in more detail. FIG. 3A shows a front perspective view of front housing 102, which may have a handle 112 (e.g. finger grip handle), drain port 114, and attachment clip 116. FIG. 3B shows a back perspective view of front housing 102, which may have molded fluid channels 118, a first portion 120a of reservoir 120, a first pump ramp or profile 122 configured and dimensioned for mating with a peristaltic pump, and a second pump ramp or profile 124 configured and dimensioned for mating with a peristaltic pump.

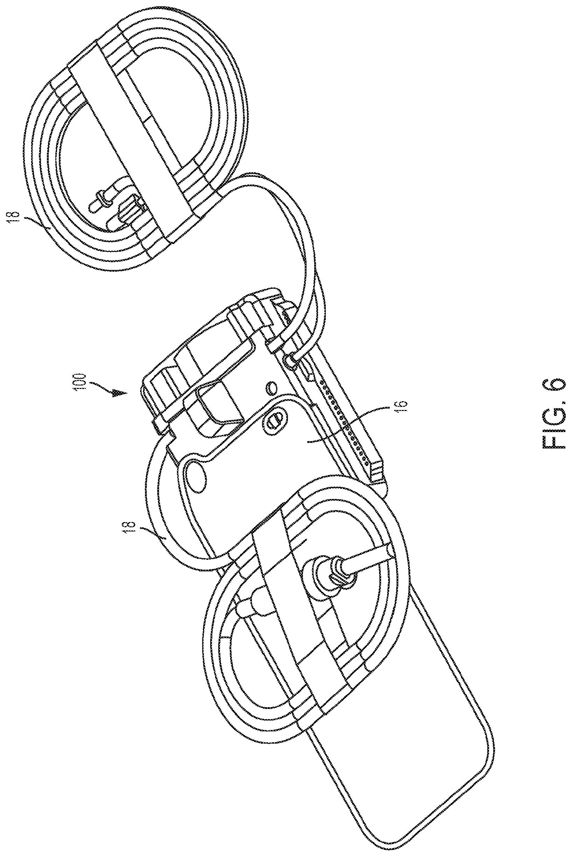

A drain bag 16 (see FIG. 6) may be attached to the front of front housing 102 via the drain port 114 and attachment clip 116 such that when surgical cassette 100 is coupled with console 14 and fluid is aspirated from an eye E of a patient P, the fluid is capable of being collected in the drainage bag 16 via drain port 114. The drain bag 16 may be supported on surgical cassette manifold 101 by attachment clip 116 and/or drain port 114.

Drain port 114 on front housing 102 of surgical cassette manifold 101 may be recessed allowing for a lower or low profile handle 112. Having a low or lower profile drain port 114 allows a drain bag (not shown) to sit flush again front housing 102. In an embodiment, placing drain port 114 substantially in the middle of the surgical cassette manifold from top to bottom moves the location of the center of mass of surgical cassette manifold 101 making the surgical cassette manifold more ergonomic. Moreover, locating drain port 114 closer to the upper/top edge of front housing 102 allows for a more compact assembly of the surgical cassette manifold and allows for better access to components/handles of console 14. See FIG. 6.

In an embodiment, a fluid channel 118 runs in a vertical direction from lower tube connection 136 (that is fluidly connected to the second tubing segment 108 that makes up the second peristaltic pump) to drain port 114 out to the drain bag 16. This molded fluid channel 118 eliminates the need for tubing.

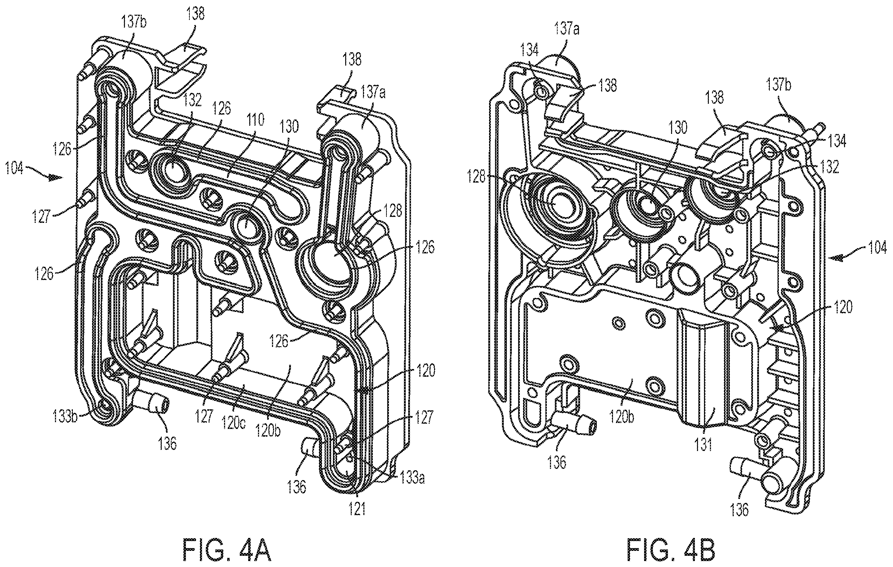

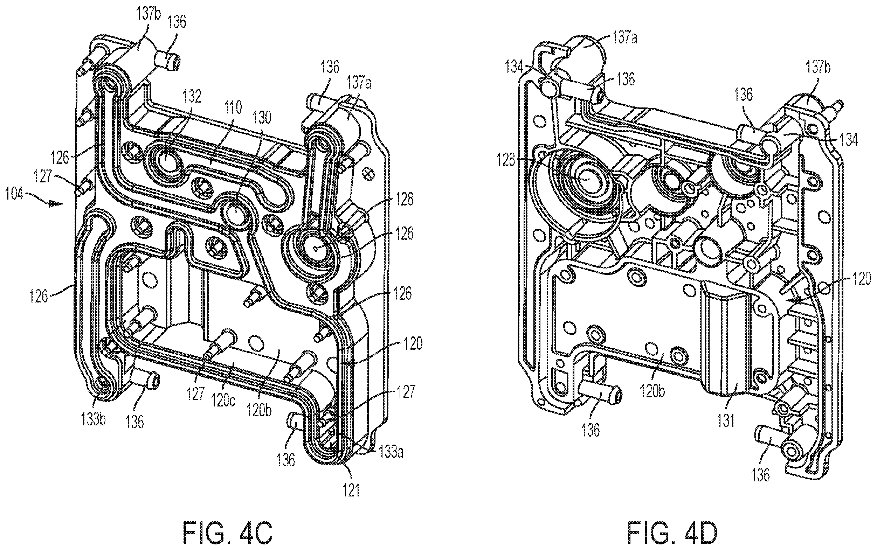

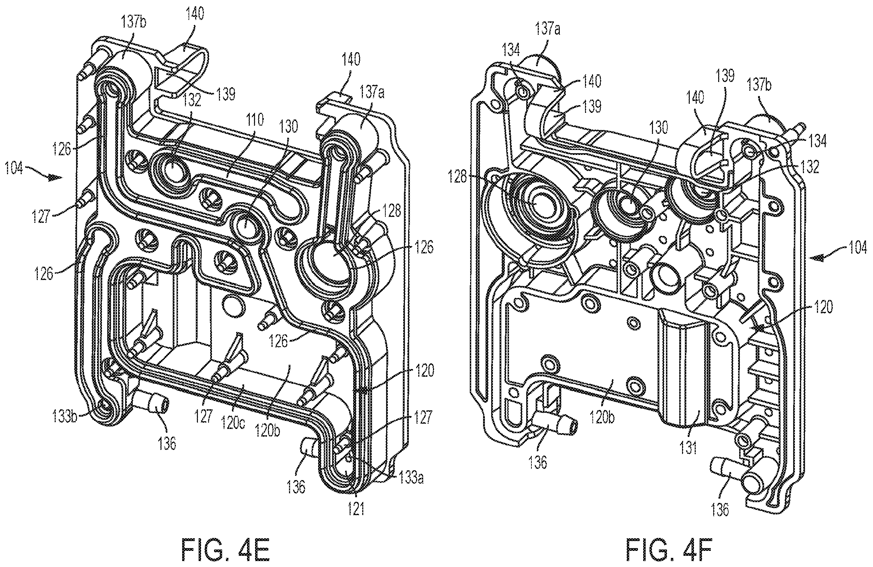

As shown in FIG. 3B, front housing 102 also may have seal channels 125, which are configured and dimensioned to mate with a seal lip 126 (shown in FIGs. 4A, 4C, and 4E) that extends outwardly or perpendicularly from the surface of gasket 110 and is a part of gasket 110 to create a seal or lid over molded fluid channels 118. The seal lip may have any dimension suitable for mating with seal channel 125. In an embodiment, seal lip 126 may be tapered, starting thicker at its proximal end and becoming thinner towards its distal end. In another embodiment, seal lip 126 may be slightly larger than seal channel 125 to create a snug fit. In a further embodiment, co-molding or over-molding gasket 110 onto rear housing 104 eliminates the potential leak path in the direction of rear housing 104. Seal lip 126 provides positioning alignment on front housing 102 and rear housing 104.

Referring to FIGs. 4A - 4F, various exemplary embodiments of rear housing 104 are shown. FIG. 4A is a front perspective view of rear housing 104 and FIG. 4B is a back perspective view of rear housing 104. As shown in FIG. 4A rear housing 104 has a gasket 110 co-molded or over-molded to it. Gasket 110 has seal lip 126 which extends away or protrudes in a substantially perpendicular direction from a plane of gasket 104 and rear housing 104. Gasket 110 may include a pressure/vacuum sensor diaphragm 128, vent valve control dome 130, and/or irrigation valve control dome 132. Vacuum/pressure sensor diaphragm 128 may be a sealed flexible annular membrane with a central magnetic coupling disk which deforms: (1) proportionally outwards under fluid pressure conditions compressing a magnetically-coupled force displacement transducer of console 14 allowing for non-fluid contact measurement of fluid pressure level inside the aspiration fluid pathways of surgical cassette manifold 101; and (2) proportionally inwards under fluid vacuum conditions extending the magnetically-coupled force displacement transducer of console 14 allowing for non-fluid contact measurement of fluid vacuum level inside the aspiration fluid pathways of surgical cassette manifold 101.

In an embodiment, gasket 110 may be molded, co-mold, or two-shot molded onto or with rear housing 104. Molding gasket 110 onto rear housing 104 in such a manner reduces or eliminates a leak path which is possible with molded fluid channels when using two different materials. In an embodiment, a method of eliminating leaking of molded fluid channels by combining two different materials for creating a proper seal is envisioned resulting in an easier manufacturing method by creating a self-aligning gasket 110. In an embodiment, when assembling rear housing 104 to front housing 102 mating of seal lip 126 and seal channel 125 can be achieved using a plurality of alignment pins 127 which mate with their counterpart pin holes 129. Using alignment pins 127 and pin holes 129 as opposed to the flexible seal lip 126 and seal channel 125 allows for an easier and more efficient assembly process. Thus, molding gasket 110 onto or with rear housing 104 results in pre-alignment/pre-keyed/pre-orientation of seal lip 126 for properly sealing molded fluid channels 118 on front housing 102, thus reducing or even eliminating leaking and increasing ease of manufacture.

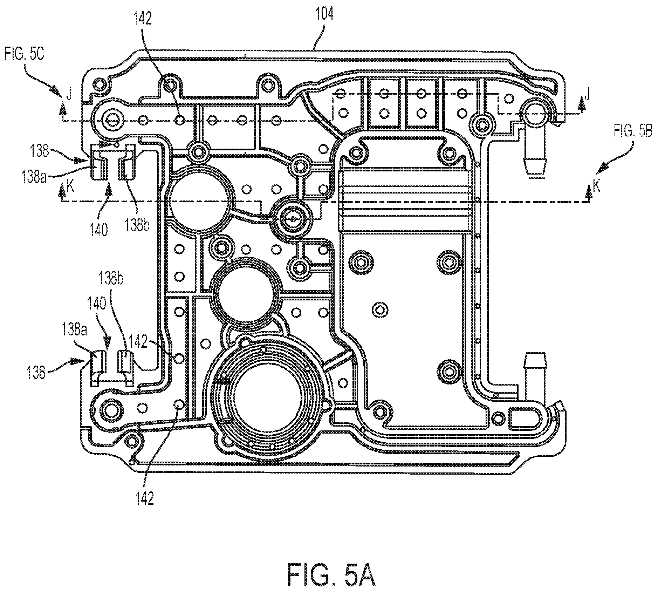

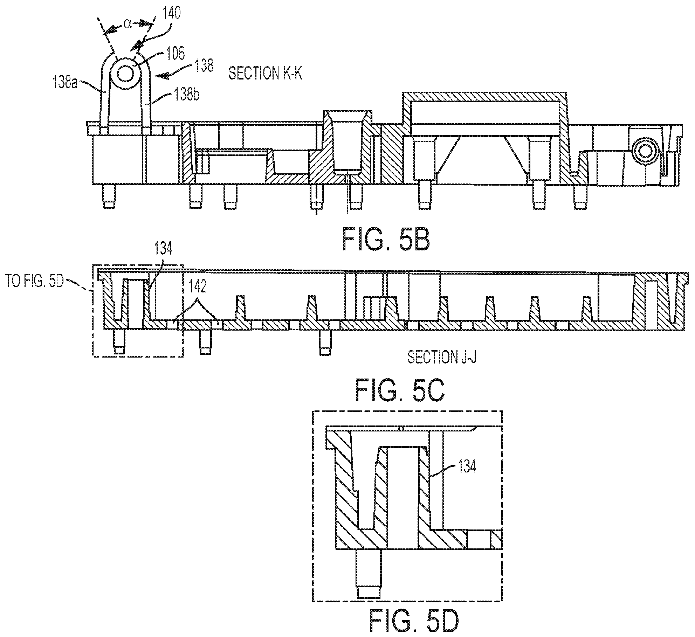

Rear housing 104 may also include a second portion 120b of reservoir 120, upper tube connections 134, lower tube connection 136, and one or more tubing retainer clips 138. In an embodiment, upper tube connections 134 have a slight taper from bottom toward the top so that the tubing stays on the upper tube connections 134. See FIG. 5D. Lower tube connection 136 may have a tapered head (as shown in FIG. 5A) to secure second tubing 108 to lower tube connections 136.

In an embodiment, second tubing 108 may have a first end and a second end that couple with lower tube connections 136. Once surgical cassette manifold 101 is assembled, second tubing 108 and ramp 124 are configured to couple with a peristaltic rollers located on console 14 (not shown) to create a peristaltic pump. In an embodiment, lower tube connections 136 are on the same axis, i.e. there is axial alignment of the inflow and outflow of the tubing 108, and maintain a specific distance apart resulting in a more accurate peristaltic pump due to the controlled length of second tubing 106, which provides a consistent flow rate and a consistent interface with ramp 124 and peristaltic rollers. Moreover, such aligned and consistent interfaces results in less noise/sound generated by the peristaltic pump during operation.

In an embodiment, reservoir 120 may have a sump 121. Sump 121 is a portion of reservoir 120 that extends below a bottom 120c of reservoir 120 that promotes fluid to flow from the reservoir 120 to sump 121 and out a lower tube connection 136 via the second tubing 108. Sump 121 (1) reduces turbulence of the tank by pulling bubbles away from the level detector housed in the console 14 that couples with window 131 for more accurate detection of the fluid in reservoir 120; and (2) ensures drain inlet port 133a fluidly connected to a lower tube connection 136 is always below fluid, therefore fluid is consistently pumped out and not air, which may cause the drain bag 16 to balloon. In an embodiment, window 131 may be a prism.

In another embodiment, the fluid level detector and window 131 are located on one side of reservoir 120 and sump 121 and baffle 135 is on the other side of reservoir 120. This configuration ensures limited or no interaction between the fluid entering and exiting the reservoir and the fluid level detector and window 131 to allow for a more precise reading of the level of fluid in reservoir 120. Moreover, the combination of baffle 135 and sump 121 provides a guide for the fluid entering reservoir 120 from molded fluid channels 118 and exiting through drain pump inlet port 133a to reduce turbulence in reservoir 120. Fluid may exit reservoir 120 via drain pump inlet port 133a via lower tube connection 136, which may be coupled with a first end of second tubing 108 and a second end of second tubing 108 may be coupled to a second lower tube connection 136 which is coupled to drain pump outlet 133b. Drain pump outlet 133b is coupled with a drain bag 16 to allow fluid to be removed from reservoir 120 via the second peristaltic pump.

Referring to FIGs. 4C and 4D, rear housing 104 is shown in an alternative embodiment with respect to how first tubing 106 couples with rear housing 104. Pump tube inlet 137a and pump tube outlet 137b couple with barbs 136, which in turn are configured and dimensioned to couple with first tubing 106. See FIG. 2b. During assembly rear housing 104 is mated with front housing 102 and first tubing 106 is configured to conform with first pump ramp or profile 122, which is configured and dimensioned for mating with a peristaltic pump located within console 14 (not shown).

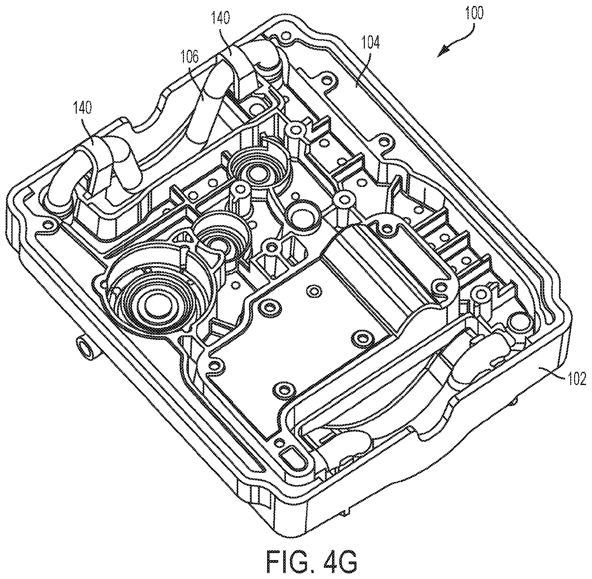

Referring to FIGs. 4E and 4F, rear housing 104 is shown in an alternative embodiment with respect to how first tubing 106 couples with rear housing 104. A first end and a second end of first tubing 106 are fed through a gap 139 of tubing catch 140 and pump tube inlet 137a and pump tube outlet 137b couple with the first end and the second end of first tubing 106 via upper tube connections 134 by placing the first end and the second end of the first tubing 106 over the upper tube connection 134, which connect to molded fluid channels 118. Other mechanisms of connecting the first and second ends of first tubing 106 to upper tube connection 134 known in the art are also contemplated. FIG. 4G shows assembled cassette 100 having the rear housing 104 embodiment shown in FIGs. 4E and 4F with first tubing 106 fed through gaps 139 of tubing catch 140 of rear housing 104.

Referring to FIGs. 5A and 5B, tubing retainer clips 138 (shown in FIGs. 4A and 4B) may have a first prong 138a and a second prong 138b creating an opening 140. Tubing retainer clips 138 protrude substantially perpendicularly from a plane of the back side of rear housing 104. In an embodiment, tubing retainer clips 138 may slightly angle towards each other as shown in FIG. 5A. Tubing retainer clips 138 are configured and dimensioned to assist with easy assembly of surgical cassette manifold 101 and maintaining first tubing 106 in a specific orientation after assembly. Tubing retainer clips 138 may be over center clips.

During assembly of surgical cassette manifold 101, a first end and a second end of first tubing 106 are coupled with upper tube connections 134 (see FIG. 4B) via pump tube inlet 137a and pump tube outlet 137b by placing the first end and the second end of the first tubing 106 over the upper tube connection 134, which may connect to molded fluid channels 118. Once the first and second end of the first tubing 106 is coupled with the upper tube connections 134, first tubing 106 may be pushed through opening 140. First tubing 140 may pushed through opening 140 between the first prong 138a and the second prong 138b by an operators hand, using tool or through an automation step with equipment. The nature of the tubing allows for it to deform under pressure and fit between the first prong 138a and second prong 138b. In an embodiment, first tubing 106 may be stretched to minimize the diameter of the tubing to enable insertion between first prong 138a and second prong 138b. Once first tubing 106 is through first prong 138a and second prong 138b, the shape and size of first prong 138a, second prong 138b, and opening 140 prevent first tubing 106 from backing back out through opening 140 after assembly. In an embodiment, each prong has an angle to help with easing first tubing 106 into opening 140 and the angle between the first prong 138a and the second prong 138b is α as shown in FIG. 5B. In an embodiment, the angle between first prong 138a and second prong 138b may be approximately 60 degrees. In another embodiment, the larger the angle the easier to insert tubing 106 into opening 140, however a 0 degree angle would not help much. In an embodiment, the angle between first prong 138a and second prong 138b may be between 30 degrees and 90 degrees.

As discussed above and shown in FIGS. 3B and 4A, reservoir 120 may be comprised of two pieces with an elastomeric seal in between the two pieces. For example, reservoir 120 may include first portion 120a and second portion 120b. In prior art tanks that are composed of multiple pieces, adhesives are used resulting in additional material for assembly. In contrast, in an embodiment of the present invention, when front housing 102 and rear housing 104 are ultrasonically welded together with gasket 110 co-molded to the rear housing, gasket 110 creates a seal around and between the edges of the first portion 120a and the second portion 120 b. In the present embodiment, the joining of the first portion 120a and the second portion 120b with the co-molded or over-molded gasket 110 results in a mechanical seal. In another embodiment, the front housing 102 and rear housing 104 may be press fit together with or without the use of adhesives to replacing ultrasonic welding. These techniques may have a cost savings advantage of avoiding extra material from a manufacturing and cost of goods perspective. In another embodiment, other mechanisms of assembly that may be used to combine front housing 102 and rear housing 104 include, but are not limited to, laser welding, a rotating latch, a snap clip latch, or fasteners, such as screws, rivets, and/or pins.

According to an embodiment, having a first portion 102a and a second portion 102b with a gasket 110 decreases the dimensional tolerance requirements for the first and second portions where the gasket portion comes in contact with the reservoir 120 due to the compressive nature of the seal. In an embodiment, gasket 110 has a seal lip 126 that mates with a seal channel 125 on second portion 102b of the reservoir tank 120 of the rear housing 104, thereby creating a seal when the front housing 102 and rear housing 104 are mated. In an embodiment, there is no need to actually displace or squeeze the gasket between the two covers to create a non-leaking seal, although some pressure may be accommodated or desired, or may result from tolerances during manufacturing. See FIG. 3B.

Referring to FIG. 5A and 5C, in an embodiment, rear housing 104 may have one or more venting/securing holes 142. The functionality of venting/securing holes 142 is two-fold. First, venting/securing holes 142 assist with venting or gas release during the co-molding or over-molding process when gasket 110 is coupled with rear housing 104. Holes 142 assist with the flow of material to minimize or eliminate the back pressure. Second, venting/securing holes 142 may be reversed tapered to help secure or increase adherence of gasket 110 to rear housing 104. For example, should the chemical reaction binding gasket 110 to rear housing 104 not occur or not completely occur, a mechanical adhesion can be achieved with the reversed taper of venting/securing holes 142. FIG. 5C shows a cross-section along J-J showing holes 142. Holes 142 may have any degree of taper suitable for securing gasket 110 to rear housing 104 and/or making molding/manufacturing easier.

In an embodiment, surgical cassette manifold 101 may be made substantially of a plastic material except for gasket 110. The plastic material may be acrylonitrile-butadiene-styrene (ABS), polycarbonate (PC), polyethylene, viton, or other rigid plastic or plastic material. In addition, the material may be such that it is transparent enabling a user to visualize various features of surgical cassette manifold 101. For example, all components may be transparent, including reservoir 120. In an embodiment, a lights emitted from console 14 may be shone through surgical cassette manifold 101 to provide a backlight and allow a user to visualize the fluid flow as it flows from handpiece 12 through molded fluid channels 118 into reservoir 120 out to the drain bag 16. In embodiment, the backlight may also be used as a surgical cassette manifold type detector.

The invention is defined in the following claims.

## Claims

1. A surgical system (10), comprising:
a cassette (100) comprising a front housing (102), a rear housing (104), and a reservoir (120), wherein the front housing comprises one or more molded fluid channels (118), and wherein the front housing, rear housing, and reservoir are made of a transparent material; and
a console (14) comprising a cassette receptacle configured and dimensioned to receive the cassette (100), wherein the cassette receptacle comprises a light,
wherein when in operation, the light is configured to illuminate the cassette enabling visualization of fluid flow through the molded fluid channels (118) and/or into the reservoir (120);
**characterised in that**
the system further comprises a cassette detector, wherein the cassette detector is configured to determine a pumping functionality of the cassette.

2. The surgical system of claim 1, wherein the pumping functionality is selected from the group consisting of peristaltic, Venturi, or both.

3. The surgical system of claim 1, wherein the cassette detector is selected from the group consisting of reflective object sensor, photo interrupter sensor, ultrasonic, laser distance sensor, bar code sensor, and pattern recognition sensor.

## Patentansprüche

1. Chirurgisches System (10), umfassend:
eine Kassette (100), umfassend ein vorderes Gehäuse (102), ein hinteres Gehäuse (104) und einen Behälter (120), wobei das vordere Gehäuse einen oder mehrere geformte Fluidkanäle (118) umfasst und wobei das vordere Gehäuse, das hintere Gehäuse und der Behälter aus einem transparenten Material hergestellt sind; und
eine Konsole (14), umfassend eine Kassettenaufnahme, die konfiguriert und bemessen ist, um die Kassette (100) aufzunehmen, wobei die Kassettenaufnahme ein Licht umfasst,
wobei das Licht, wenn in Betrieb, konfiguriert ist, um die Kassette zu beleuchten, wobei eine Visualisierung einer Fluidströmung durch die geformten Fluidkanäle (118) und/oder in den Behälter (120) ermöglicht wird;
**dadurch gekennzeichnet, dass**
das System ferner einen Kassettendetektor umfasst, wobei der Kassettendetektor konfiguriert ist, um eine Pumpfunktionalität der Kassette zu bestimmen.

2. Chirurgisches System nach Anspruch 1, wobei die Pumpfunktionalität aus der Gruppe ausgewählt ist, bestehend aus peristaltisch, Venturi oder beidem.

3. Chirurgisches System nach Anspruch 1, wobei der Kassettendetektor ausgewählt ist aus der Gruppe, bestehend aus Reflexionsobjektsensor, Fotounterbrechersensor, Ultraschall, Laser-Abstandssensor, Strichcodesensor und Mustererkennungssensor.

## Revendications

1. Système chirurgical (10), comprenant :
une cassette (100) comprenant un boîtier avant (102), un boîtier arrière (104) et un réservoir (120), dans lequel le boîtier avant comprend un ou plusieurs canaux de fluide moulés (118), et dans lequel le boîtier avant, le boîtier arrière et le réservoir sont constitués d'un matériau transparent ; et
une console (14) comprenant un réceptacle de cassette conçu et dimensionné pour recevoir la cassette (100), dans lequel le réceptacle de cassette comprend une lumière,
dans lequel, en fonctionnement, la lumière est configurée pour éclairer la cassette permettant la visualisation de l'écoulement de fluide à travers les canaux de fluide moulés (118) et/ou dans le réservoir (120) ;
**caractérisé en ce que**
le système comprend en outre un détecteur de cassette, dans lequel le détecteur de cassette est configuré pour déterminer une fonctionnalité de pompage de la cassette.

2. Système chirurgical selon la revendication 1, dans lequel la fonctionnalité de pompage est choisie dans le groupe constitué par péristaltique, Venturi, ou les deux.

3. Système chirurgical selon la revendication 1, dans lequel le détecteur de cassette est choisi dans le groupe constitué de capteur d'objet réfléchissant, capteur photo-interrupteur, capteur à ultrasons, capteur de distance laser, capteur de code-barres et capteur de reconnaissance de formes.
